# EUROPEAN PATENT SPECIFICATION

(11) **EP 2 117 555 B1**
(45) Date of publication and mention of the grant of the patent: **17.10.2012**
(21) Application number: 07822466.4
(22) Date of filing: 09.11.2007
(51) Int. Cl.: A61K 31/685, A61K 45/06, A61K 31/4965, A61K 31/513, A61K 31/52, A61K 31/7048, A61P 35/02

(54) **USE OF TRI-SUBSTITUTED GLYCEROL COMPOUNDS FOR THE TREATMENT OF HEMATOLOGICAL MALIGNANCIES**
VERWENDUNG VON TRISUBSTITUIERTEN GLYCERIN-VERBINDUNGEN ZUR BEHANLDUNG VON BÖSARTIGEN BLUTKRANKHEITEN
UTILISATION D'UN COMPOSÉ DE GLYCÉROL TRI-SUBSTITUÉ DANS LE TRAITEMENT DES HÉMOPATHIES MALIGNES

(30) Priority: 20.12.2006 US 875963 P
(43) Date of publication of application: 18.11.2009
(73) Proprietor: Universitätsklinikum Hamburg-Eppendorf, 20246 Hamburg (DE); Alphaptose Gmbh, 20149 Hamburg (DE)
(72) Inventor: ZANDER, Axel Rolf, 22359 Hamburg (DE); AYUKETANG, Francis Ayuk, 22767 Hamburg (DE); RICHTER, Wolfgang, 81243 München (DE); WEBER, Lutz, 82110 Germering (DE)
(74) Representative: Huenges, Martin
(86) International application number: PCT/EP2007/062178
(87) International publication number: WO 2008/074572

(56) References cited:
- WO-A-01/74333
- WO-A-97/04765
- GB-A- 1 583 661
- US-B1- 6 180 137
- GAJATE C ET AL: "The antitumor ether lipid ET-18-OCH3 induces apoptosis through translocation and capping of Fas/CD95 into membrane rafts in human leukemic cells" 15 December 2001 (2001-12-15), BLOOD, W.B.SAUNDERS COMPANY, ORLANDO, FL, US, PAGE(S) 3860-3863 , XP002233039 ISSN: 0006-4971 abstract
- MOLLINEDO F ET AL: "SELECTIVE INDUCTION OF APOPTOSIS IN CANCER CELLS BY THE ETHER LIPID ET-18-OCH3 (EDELFOSINE): MOLECULAR STRUCTURE REQUIREMENTS, CELLULAR UPTAKE, AND PROTECTION BY BCL-2 AND BCL-XL" CANCER RESEARCH, AMERICAN ASSOCIATION FOR CANCER RESEARCH, BALTIMORE, MD, US, vol. 57, no. 7, 1 April 1997 (1997-04-01), pages 1320-1328, XP001204170 ISSN: 0008-5472

## Description

The present invention relates to the use of a tri-substituted glycerol compound in combination with at least one other medicament comprising one or more additional active ingredients which is/are selected from the group consisting of antimetabolites, plant alkaloids, inhibitors of topoisomerase II and proteasome inhibitors for use in the treatment of hematological malignancies. The invention also refers to a kit-of parts comprising the combination of such medicaments. Finally, the invention also relates to a corresponding method for the treatment of such conditions as well as to an *in vitro* method for determining the susceptibility of such malignant cells to medicaments as defined in the invention.

Hematological malignancies such as leukemia and lymphoma are the types of cancer that affect blood, bone marrow and lymph nodes. Chromosomal translocations strongly influence the etiology of these diseases, while this is uncommon in solid tumors. This leads to a different approach in diagnosis and treatment of hematological malignancies (see, e.g., Dewald, G.W. et al. (1995) Semin. Oncol. 22, 341-354; Grimwade, D. et al. (1998) Blood 92, 2322-2333; Jaffe, E.S. et al. (2001) Tumors of hematopoietic and lymphoid tissue. International Agency for Research on Cancer, IARC Press, Lyon, France; Grimwade, D. et al. (2004) Ann. Hematol. 83, Suppl. 1, S45-S48).

Many patients with disseminated hematological malignancies are treated with chemotherapy (optionally in combination with irradiation) at some point during their illness. While response rates are generally high in some malignancies, such as lymphoid malignancies, only a subset of patients will be cured. Even amongst the lymphoid malignancies some (e.g. chronic lymphocytic leukemia (CLL), stage III/IV follicular small cleaved cell lymphoma, stage III/IV marginal zone lymphoma, stage III/IV mantle cell lymphoma, and multiple myeloma) are currently incurable with standard dose chemotherapy.

However, the currently available non-surgical therapeutic options are associated with adverse drug reactions, some of which are severe, which represents a limiting factor especially for chemotherapeutic approaches for an increasingly aging population with multiple pathologies.

One problem to be addressed in the treatment of such malignancies that recurrence of these tumors is unavoidable even with, or despite, the use of aggressive therapeutic regimen. Long-term therapy with chemotherapeutics is not possible because of the toxicity of these substances, and repeated use is not worthwhile because the response rates then approach zero. The limit for radiation exposure is likewise reached relatively quickly, if it is intended to treat even the primary tumor effectively. Thus, the situation is still such that after completion of the first therapeutic regimen it is necessary to "wait" until there has been renewed occurrence.

Presently, the chemotherapies for the treatment of hematological malignancies involve the administration of one or more anti-neoplastic agents (i.e. cytostatic and/or cytotoxic chemotherapeutics) such as alkylating agents, plant alkaloids, antimetabolites, antibiotics, inhibitors of topoisomerasse I and/or II, inhibitors of tyrosine kinases, proteosome inhibitors, and steroids (cf., for example, Alexanian, R. et al. (1969) JAMA 208, 1680-1685; Rai, K.R. et al. (1981) Blood 58, 1203-1212; Alexanian, R. et al. (1990) Am. J. Hematol. 33, 86-89; Bassan, R. et al., 1992, Leukemia 6 (suppl. 2), 186-190; Keating, M.J. (1993) Chemotherapy of chronic lymphocytic leukemia, in: Cheson, B.D. (ed.) Chronic Lymphocytic Leukemia. Scientific Advances and Clinical Developments. Marcel Dekker, New York, NY, p. 297 ff.; Boulard, F. et al. (1993) Cancer Invest. 11, 534-553; Smith, M. et al. (1996) J. Clin. Oncol. 14, 18-24; Noordijk E. et al. (1997) Int. J. Radiat. Oncol. Biol. Phys. 39, 173; Miller, T.P. et al. (1998) N. Eng. J. Med. 339, 21-26; Drucker, B.J. (2001) N. Engl. J. Med. 344, 1031-1037; Richardson, P.G. et al. (2002) ASCO Annual Meeting Proceedings, 11a). All common anti-neoplastic agents have to be administered systematically, as they represent molecules also exerting nonselective effects on their physiological targets. However, this implies that such anti-neoplastic (cytostatic) agents, especially if applied over a long period of time, will have a harmful effect on the body's normal cells, i.e. its use is associated with significant adverse side effects mainly affecting the fast dividing body cells. Common side effects include *inter alia* genetic damage, nausea, vomiting, diarrhea, constipation, hair loss, immune suppression, myelo-suppression, anemia, hemorrhage, cytotoxicity, secondary neoplasms, and the like).

Thus, there is still a need either for alternative chemotherapeutics displaying less adverse side effects while preserving their pharmacological efficacy or for any compounds to be administered in combination with the above anti-neoplastic agents, wherein the adjunction results in a synergistic cytostatic and/or cytotoxic effect on malignant cells as compared to an individual application. Such adjunction would again allow administering a reduced dosage of anti-neoplastic agents to achieve the same therapeutic efficacy thus resulting in less severe adverse side effects.

Tri-substituted glycerol compounds belonging to the class of synthetic ether-linked alkyl-lysophospholipids might be candidate compounds for such an combinatorial approach.

Such synthetic ether-linked alkyl-lysophospholipids are known to have an anti-cancerogenic activity (reviewed, e.g., by Arthur, G., and Bittman, R. (1998) Biochim. Biophys. Acta 1390, 85-102; Jendrossek, V., and Handrick, R. (2003) Curr. Med Chem. Anti-Canc. Agents 3, 343-353; Mollinedo, F. et al. (2004) Curr. Med. Chem. 11, 3163-3184). 1-*O*-octadecyl-2-*O*-methyl-glycero-3-phosphocholine (also referred to as ET-18-OCH3, AP-121 or edelfosine) is considered to be the prototype of these lipids. 1-*O*-octadecyl-2-*O*-methyl-glycero-3-phosphocholine represents a synthetic analogue of the platelet activating factor (PAF; 1-*O*-alkyl-2-acetyl-sn-glycero-3-phosphocholine), a potent phospholipid activator and mediator of many leukocyte functions, including platelet aggregation, inflammation, and anaphylaxis. Unlike most conventional chemotherapeutic drugs, these synthetic ether lipids do not directly target cellular DNA but rather affect the plasma membrane lipid composition and/or interfere with various signal transduction pathways. Thus, their mode of action does not depend on the presence of particular cellular receptors or is it cell cycle-dependent.

Cancer chemotherapy generally aims to slow the growth of, or destroy, cancer cells while avoiding collateral damage to surrounding cells and tissues. Consequently, the most effective anticancer agents are those that are able to selectively target cancer cells while leaving normal cells relatively unaffected. Synthetic ether-lipids have been shown to exert such an effect (cf., for example, Magistrelli, A. et al. (1995) Drug. Metab. Dispos. 23, 113-118). Several mechanisms of action have been proposed for the toxicity of ether-lipids towards cancer cells, including the cells' lack of alkyl cleavage enzymes. The resultant inability to hydrolyze the ether-lipids leads to their intracellular accumulation and to consequent damage to cell membrane lipid organization. Other potential mechanisms of ether-lipid action include effects on levels of intracellular protein phosphorylation, and disruption of cellular lipid metabolism. Normal cells typically possess the means to avoid or overcome the potentially toxic effects of ether-lipids, while cancer cells do not.

Thus far, synthetic ether lipids have been used for the treatment of different types of solid tumors such as brain tumors or mamma carcinomas (cf., for example, the German Patent DE 2619686 as well as the International Patent Applications WO 99/59599 and WO 00/01392, respectively). GB 1 583 661 describes the use of synthetic lysolecithin compounds for increasing resistance to diseases and tumors. US 6 180 137 describes the use of etherlipids in the treatment of cancers and inflammatory disorders. WO 97/04765 discloses the use of inhibitors of CoA-independent transacylase for inhibition or reducing cell proliferation. WO 01/74333 describes the use of L or D steroisomer of an ether lipid (as anti-cancer or anti-inflammatory agents). Gajate, C. et al. (2001) Blood 98, 3860-3863 shows that ether lipid ET-18-OCH₃ induces apoptosis. However, although the anti-tumor activity of these synthetic ether lipids has been experimentally proven in several animal tumor models, their clinical use is often hampered by systemic cytotoxic effects including hemolysis, particularly in the gastrointestinal tract but also *inter alia* in lung, liver or kidney.

Currently, in the vast majority of clinical trials on synthetic ether lipids the compounds are administered to patients orally or by using the intravenous route. In this context, it was found that the intravenous administration of a liposomal formulation and a lipophilic oil-in-water emulsion, respectively, is advantageous as compared to the free compound in order to improve therapeutic efficacy while reducing nonspecific toxicity *in vivo* (see, for example, Ahmad, I. et al. (1997) Cancer Res. 57, 1915-1921 as well as International Patent Application WO 91/09590).

However, it is also known in the art that certain ether phospholipid and carbamoyl salts while exhibiting benefits to a patient as competitive inhibitors of PAF or tumor growth with single or repeated injections, cause detrimental effects in the area of the injection. These detrimental effects are evident as lysis of red blood cells, severe edema, inflammation, and injection site-necrosis. These adverse effects are also called "detergent" effects. Where repeated injections are required, these detrimental effects are particularly disadvantageous as they render the sites of administration unsuitable and require fresh sites. Since the number of suitable sites on a patient is limited, it would be highly desirable to avoid said detrimental effects associated with intravenous administration of 1-*O*-octadecyl-2-*O*-methyl-glycero-3-phosphocholine.

Rather recently, it has been shown that in order to limit systemic side effects it is also possible to administer synthetic ether lipids orally together with a liquid drinkable vehicle. In the International Patent Application WO 99/59599, it is described that 1-*O*-octadecyl-2-*O*-methyl-glycero-3-phosphocholine can be administered together with water- or milk-based vehicles containing at least 3% (w/w) fat and/or protein. It is tempting to speculate that an efficient binding of 1-*O*-octadecyl-2-*O*-methyl-glycero-3-phosphocholine to the proteins and/or other lipids "mask" the ether-lipid thus resulting in a reduction of adverse side effects.

Nevertheless, in 10-20% of the patients treated with such water- and/or milk-based vehicles significant gastrointestinal incompatibilities (corresponding to WHO toxicity grades III and IV, respectively) have been observed that are associated with loss of appetite, nausea and/or vomiting, diarrhea, constipation or the like (see, for example, Drings, P. et al. (1992) Onkologie 15, 375-382).

Thus, there remains a need for a medicament for the treatment of hematological malignancies that overcomes the above limitations. In particular, there is a need for a medicament, which allows for an easy and convenient administration and provides the required pharmaceutical efficacy without significant side effects.

Accordingly, it is an object of the present invention to provide such a medicament for the treatment of hematological malignancies.

This object is achieved by the use of a tri-substituted glycerol compound having the features of independent claim 1 for the manufacture of a corresponding medicament that can be used in combination with one or more other pharmaceuticals. Some of the preferred embodiments of the present invention are defined by the subject matter of the dependent claims.

It has been found that tri-substituted glycerol compounds such as 1-*O*-octadecyl-2-*O*-methyl-glycero-3-phosphocholine are not only suitable for the use as a medicament for the selective treatment of hematological malignancies but also enhance the cytostatic and/or cytotoxic effects of several known chemotherapeutics on malignant cells in a synergistic manner. Thus, these synergistic effects allow for the administration of reduced drug doses in order to achieve the same pharmaceutical efficacy as compared to an individual administration, which will again reduce the risk of observing systemic adverse side effects.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%.

In a first aspect, the present invention relates to the use of a tri-substituted glycerol compound according to formula (I) or an enantiomer, diastereomer or pharmaceutically acceptable salt thereof for the manufacture of a medicament for the treatment of hematological malignancies, 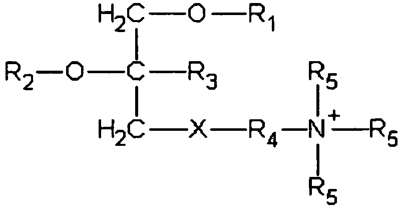 wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl;
R₃ is selected from the group consisting of hydrogen and C₁-C₃ alkyl;
R₄ is selected from the group consisting of C₁-C₃ alkyl and C₃-C₆ cycloalkyl; and
R₅ is selected from the group consisting of hydrogen and methyl
and wherein the medicament is a pharmaceutical solid dosage form for oral administration and is used in combination with at least one other medicament comprising one or more additional active ingredient/s which is/are selected from the group consisting of antimetabolites, plant alkaloids, inhibitors of topoisomerase II and proteasome inhibitors.

The tri-substituted glycerol compound may be used in amorphous or in crystalline form. The term "amorphous", as used herein, refers to a solid in which there is no long-range order of the positions of the atoms, i.e. a non-crystalline material. In preferred embodiments, the tri-substituted glycerol compound is present in crystalline form.

The terms "Cₙ alkyl", "Cₙ hydroxyalkyl", and "Cₙ cycloalkyl", as used herein, denote an alkyl group, a hydroxyalkyl group or a cycloalkyl group having n carbon atoms, respectively. For example, the term "C₁₈ alkyl" refers to an alkyl group having 18 carbon atoms. The alkyl groups or hydroxyalkyl groups according to the invention may be straight or branched.

The tri-substituted glycerol compounds of formula (I) have one or more asymmetric centers and thus they can exist as enantiomers or diastereomers. Accordingly, the medicament used in the present invention may comprise either one or more separate individual isomers (such as the L form and the D form) or mixtures of isomers, preferably racemic mixtures.

The tri-substituted glycerol compounds of formula (I) may be comprised in the medicament as pharmaceutically acceptable salts. Such salts may comprise any pharmaceutically acceptable anion "neutralizing" the positive charge of the nitrogen (e.g. chloride, bromide or iodide) or any pharmaceutically acceptable cation "neutralizing" the negative charge of the phosphate or sulfate moiety (e.g. sodium or potassium cations).

In a particular preferred embodiment of the present invention, the medicament comprises a tri-substituted glycerol compound according to formula (I), wherein X is phosphate, R₁ is -(CH₂)₁₇-CH₃, R₂ is CH₃, R₃ is H, R₄ is -(CH₂)₂-, and R₅ is CH₃.

The medicament may be any pharmaceutical dosage form that is therapeutically effective. Examples of such pharmaceutical dosage forms include *inter alia* tablets, pills, capsules, suspensions, emulsions, injection or infusion solutions, tinctures, powders and the like.

The medicaments comprise at least one pharmaceutically acceptable excipient. The term "pharmaceutically acceptable excipient", as used herein, can be any substance used for the preparation of pharmaceutical dosage forms such as coating materials, film-forming materials, fillers, disintegrating agents, release-modifying materials, carrier materials, diluents, binding agents and other adjuvants, all of them well known in the art (cf. the references cited below). Preferably, the excipient comprises at least one filler, at least one binder, at least one disintegrating agent, at least one flowability-controlling agent, and at least one lubricant.

The medicament may be administered via any parenteral or non-parenteral route. Parenteral application methods comprise, for example, intracutaneous, subcutaneous, intramuscular or intravenous injection and infusion techniques. Non-parenteral delivery modes include, for instance, oral or topical administration. Furthermore, the medicament may be administered locally or systemically.

Preferably, the medicament is a solid dosage form, particularly preferably a pharmaceutical solid formulation suitable for oral application. Examples of such dosage forms include *inter alia* tablets, pills, capsules, granulates, pellets, powders, multi-particulate formulations (e.g., beads, granules or crystals), and dragees. The unit doses of multi-particulates may be incorporated into a pharmaceutical solid dosage formulation, e.g. via compression or shaping into tablets or by placing a requisite amount inside a gelatin capsule.

All these solid dosage forms for oral application as well as methods for their preparation are well established in the art (see, e.g., Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Stricker, H. (2003) Arzneiformenentwicklung, Springer Verlag, Berlin, Germany; Niazi, S.K. (2004) Handbook of Pharmaceutical Manufacturing Formulations, CRC Press, Boca Raton, FL).

In preferred embodiments, the pharmaceutical solid dosage form is selected from the group consisting of tablets, pills, capsules, and granules, with tablets being particularly preferred.

In another preferred embodiment, the solid dosage form is an enteric dosage form. That is, the dosage form remains stable in the stomach, i.e. in a highly acidic environment, with pH values in the range of ≤ 2.5. This may be achieved by providing a solid dosage form comprising a film coating. For example, the inventive dosage form may be in the form of a so-called film tablet.

Methods for the preparation of film coated dosage forms are also well established in the art (see, for example, Gennaro, A.L. and Gennaro, A.R. (2000) Remington: The Science and Practice of Pharmacy, 20th Ed., Lippincott Williams & Wilkins, Philadelphia, PA; Ritschel, W.A. & Bauer-Brandl, A. (2002) Die Tablette: Handbuch der Entwicklung, Herstellung und Qualitätssicherung. Editio-Cantor Verlag, Aulendorf, Germany; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL). Furthermore, the skilled artisan also knows how to provide film coatings with specific properties, like enteric coatings, film coating which dissolve upon contact with body fluids, controlled release coatings, taste-masking coatings or disintegrating coatings. In a particularly preferred embodiment, the solid dosage form of the invention comprises an enteric coating.

It is to be understood that the tri-substituted glycerol compound is present in the medicament in any amount being effective to achieve the desired pharmacological effect such as to stop tumor progression or to induce an apoptotic effect in tumor cells when administered to a patient. Effective amounts are generally chosen in accordance with a number of factors, e.g., the age, size and general condition of the patient and the medical condition being treated, and determined by a variety of means, for example, dose ranging trials, well known to, and readily practiced by persons of ordinary skill in art given the teachings of this invention.

Typically, in the medicament the amount of the tri-substituted glycerol compound according to formula (I) is less than 400 mg, preferably it is in the range of 30 to 250 mg, and most preferably it is in the range of 50 to 150 mg. In particularly preferred embodiments, the amount of the tri-substituted glycerol compound according to formula (I) in the medicament is 75 mg and 100 mg, respectively.

The daily dosage of the tri-substituted glycerol compound in the medicament administered to a patient is less than 1200 mg, typically less than 900 mg, preferably in the range of 30 to 600 mg, more preferably in the range of 40 to 400 mg, and most preferably in the range of 50 to 350 mg. In specific embodiments, the daily dosage is 75, 100, 150, 200, 225, and 300 mg. Preferably, the daily dosage of the tri-substituted glycerol compound is administered as a single dose such as in form of one up to four tablets or capsules. However, it may also be possible to administer the compound in multiple doses such as two or three individual doses administered during the day, e.g. in the morning, at noon, and at night.

The term "hematological malignancies" (also referred to as "hematological neoplasms"), as used herein, denotes any type of cancer that affects blood, bone marrow or lymphatic organs. Hematological malignancies include *inter alia* leukemia, lymphoma, multiple myeloma, and myelodysplastic syndrome.

The term "leukemia", as used herein, refers to any cancer of the blood or bone marrow characterized by an abnormal proliferation of blood cells, usually of white blood cells (i.e. leukocytes). Within the scope of the present invention, the term includes any acute and chronic forms of leukemia as well as any form of lymphoid or myeloid leukemia. Forms of lymphoid (or lymphocytic) leukemia are characterized in that lymphoid cells (that is, agranulocytes) such as lymphocytes and monocytes are affected, whereas when myeloid cells (that is, granulocytes) such as eosinophils, neutrophils, and basophils are affected, the disease is referred to as myeloid (or myelogenous) leukemia. In preferred embodiments, the leukemia is selected from the group consisting of acute myeloid leukemia (AML), acute lymphoid leukemia (ALL), chronic myeloid leukemia (CML), and chronic lymphoid leukemia (CLL).

The term "lymphoma", as used herein, refers to any form of cancer originating from lymphocytes, that is it includes Hodgkin lymphoma characterized by the orderly spread of the disease from one lymph node and the presence of Reed-Sternberg cells as well as to any type of non-Hodgkin lymphoma. In preferred embodiments, the hematological malignancy is selected from the group consisting of Hodgkin lymphoma and non-Hodgkin lymphoma.

The term "multiple myeloma" (also referred to as "myeloma" or "plasmacytoma"), as used herein, denotes a type of cancer of plasma cells, i.e. the immune cells in bone marrow that produce antibodies.

The term "myelodysplastic syndrome" (formerly known as "pre-leukemia"), as used herein, denotes a diverse collection of hematological conditions united by ineffective production of blood cells and varying risks of transformation to acute myelogenous leukemia. Anemia requiring chronic blood transfusion is frequently present. Although not a true malignant neoplasm, myelodysplastic syndrome is nevertheless classified within the hematological neoplasms.

The medicament may be used for the treatment of hematological malignancies in combination with at least one other pharmaceutical comprising at least one additional active ingredient. That is a medicament comprising a tri-substituted glycerol compound is used together with at least one other pharmaceutical comprising one or more additional active ingredients, i.e. active ingredients different from the tri-substituted glycerol compounds as defined in claim 1. In embodiments of the disclosure, the one or more additional active ingredients are selected from the group consisting of anti-neoplastic agents and/or from the group consisting of antibodies directed against one or more epitopes on the cell surface of hematological (hematopoetic) cells.

The term "anti-neoplastic agents" (herein also referred to as "cytostatic drugs"), as used herein, refers to any pharmaceuticals which interfere with cellular karyokinesis (i.e. mitosis) and/or cytokinesis (i.e. cell division), in particular in malignant cells (i.e. tumor cells), that is which cause an interruption, a delay or a prohibition of said process(es). Also included herein are cytostatic drugs having a cytotoxic effect as well, that is pharmaceuticals causing cell death by interfering with karyokinesis and/or cytokinesis.

Examples of anti-neoplastic agents (cytostatic drugs) that can be used in the present disclosure include *inter alia* alkylating agents, antimetabolites, plant alkaloids, antibiotics, inhibitors of topoisomerases I and/or II, steroids, inhibitors of tyrosine kinases, proteosome inhibitors, and DNA intercalating agents.

The term "alkylating agents", as used herein, denotes compounds being capable of adding alkyl groups to various electronegative groups present in cellular molecules such as nucleic acids or proteins. Typically, these compounds interfere with tumor growth and progression by cross-linking guanine nucleobases in DNA strands. This modification prevents DNA uncoiling and strand separation, and thus DNA replication.

In embodiments of the disclosure, the alkylating agents are selected from the group of nitrogen mustards, nitrosoureas, platinum derivatives, and alkylsulfonates. Examples of nitrogen mustards include *inter alia* chlorambucil (CLL), cyclophosphamide (lymphomas), melphalan (multiple myeloma), and mechlorethamine (Hodgkin lymphomas). An example of nitrosoureas is *inter alia* carmustine (lymphomas), and examples of platinum derivatives include *inter alia* cisplatin (lymphomas) and carboplatin (lymphomas, ALL). Examples of alkylsulfonates include *inter alia* busulfan (CML), and treosulfan (lymphomas).

The term "antimetabolite", as used herein, refers to chemical compounds having a similar structure as a substance (a metabolite) that is required for cellular biochemical reactions, yet different enough to interfere with the normal cellular function.

In preferred embodiments, the antimetabolites are selected from the group consisting of methotrexate, (ALL), purine analogs, and pyrimidine analogs. Examples of purine analogs include *inter alia* fludarabine (non-Hodgkin lymphomas, AML), clofarabine (ALL), pentostatine (CLL), and cladribine (CLL), and examples of pyrimidine analogs include *inter alia* cytarabine (leukemia, non-Hodgkin lymphomas), and decitabine (myelodysplastic syndrome).

Examples of plant alkaloids (i.e. naturally occurring or synthetically produced plant amines) include *inter alia* vincristine (non-Hodgkin lymphoma, ALL) and vinblastine (Hodgkin lymphomas). Exemplary antibotics that can be used in the invention include *inter alia* mitoxantrone (AML), epirubicin (lymphomas), doxorubicin (Hodgkin lymphoma), daunorubicin (AML, ALL), and bleomycin (Hodgkin lymphoma). Topoisomerase inhibitors interfere with normal function of topoisomerases type I and/or type II, that is enzymes that act on DNA topology. Topoisomerase II inhibitors such as etoposide (lymphomas, AML, CML) or teniposide (ALL) are preferred. Further anti-neoplastic agents include *inter alia* the steroid dexamethasone (non-Hodgkin lymphomas), the tyrosine kinase inhibitor imatinib (CML), the proteasome inhibitor bortezomib (multiple myeloma), and the DNA intercalating agents amsacrine (ALL, AML), and thalidomide (multiple myeloma).

The respective primary medical use of the above anti-neoplastic agents is indicated in brackets. However, this does not preclude the use of any of these drugs for the treatment of any other hematological malignancies. Furthermore, it is also possible to use a combination of two or more of these drugs for the treatment of any hematological malignancy.

In a particular preferred embodiment, the anti-neoplastic agents are selected from the group consisting of chlorambucil, cyclophosphamide, melphalan, mechlorethamine, carmustine, cisplatin, carboplatin, busulfan, treosulfan, methotrexate, fludarabine, clofarabine, pentostatine, cladribine, cytarabine, decitabine, vincristine, vinblastine, mitoxantrone, epirubicin, doxorubicin, daunorubicin, bleomycin, etoposide, teniposide, dexamethasone, imatinib, bortezomib, amsacrine, and thalidomide.

The antibodies described herein comprise monoclonal and polyclonal antibodies directed against one or more epitopes on the cell surface of hematological (hematopoetic) cells. The term "hematological (hematopoetic) cells", as used herein, denotes any cells of hematopoetic origin including cells of myeloid and lymphatic origin. The term "directed against one or more epitopes on the cell surface", as used herein, refers to the fact that an antibody used in the present invention may recognize/bind one or more cell surface molecules (i.e. epitopes) of a hematological cell.

In embodiments of the disclosure, the antibody is a monoclonal antibody, that is an antibody directed against a specific antigen that is generated from a population of B-cell lymphocytes that are all duplicates descended from one original antibody producing cell. The antibody may be a human antibody or a chimeric (humanized) antibody, for example a chimeric murine/human antibody. Methods for the generation of such "engineered" monoclonal antibodies are well established in the art (reviewed, e.g., in Coligan, J.E. et al. (2002) Current Protocols in Immunology, Wiley & Sons, Hoboken, NJ)

Examples of such monoclonal antibodies that can be employed include *inter alia* alemtuzumab (anti-CD52; CLL, lymphomas), rituximab (anti-CD20; CLL, non-Hodgkin-lymphoma), gentuzumab ozogamizin (anti-CD33; AML)

The present disclosure relates to a corresponding method for the prevention and/or the treatment of hematological malignancies, wherein the method comprises:
(a) administering to a patient a medicament or a combination of medicaments as defined in the invention.

As outlined above, the combination of medicaments may be administered via any parenteral or non-parenteral route. The individual pharmaceuticals may be administered via the same or via different routes. The medicament comprising the tri-substituted glycerol compound is administered orally, as a solid dosage form. Furthermore, the medicament is preferably administered as a single dose such as in form of one tablet or capsule per day. However, it may also be possible to administer the medicament in multiple doses such as two or three individual doses administered during the day. In case a combination of medicaments is used (such as in form of a kit-of-parts), the individual pharmaceuticals (i.e. the medicament comprising the tri-substituted glycerol compound and the at least one other pharmaceutical) may be administered simultaneously, separately or sequentially. For example, it may be possible to administer one medicament during a meal, and another one or more hours later.

In an embodiment of the disclosure, the method further comprises:
(b) determining the extent of Fas receptor gene expression in one or more malignant cells of the patient to be treated before administering the medicament or the combination of medicaments.

As already outlined above, the cell death receptor Fas (also known as APO-1 or CD95) has been identified as a cellular target of 1-O-octadecyl-2-O-methyl-glycero-3-phosphocholine (cf., for example, Gajate, C. et al. (2004) J. Exp. Med. 200, 353-365; Nieto-Miguel, T. et al. (2006) J. Biol. Chem. 281, 14833-14840). In the context of the present invention, it has been found that malignant hematological cells (i.e. tumor cells) over-expressing the Fas receptor display an increased sensitivity towards the treatment using a medicament as defined in the claims.

The terms "expression" or "gene expression", as used herein, relate to the entirety of regulatory pathways converting the information encoded in the nucleic acid sequence of a gene first into messenger RNA (mRNA) and then to a protein. Accordingly, the expression of a gene comprises its transcription into a primary hnRNA, the processing of this hnRNA into a mature RNA and the translation of the mRNA sequence into the corresponding amino acid sequence of the protein.

As used herein, the term "over-expressing" refers to an expression level of at least 100% of that of a non-malignant (non-tumorgenic) control cell (i.e. a wild-type cell). Accordingly, in one embodiment of the invention, the method further comprises comparing results of the measurements obtained in the tumor cells with those obtained in one or more control cells of a healthy subject.

The term "determining" refers to any method to detect a "gene expression product". Within the scope of the present invention, the term "gene expression product" refers not only to the protein encoded by that gene but also to the respective mRNA, which may be regarded as the "first gene product" during the course of gene expression. These methods comprise established standard procedures well known in the art (cf., for example, Sambrook, J. et al. (1989) Molecular Cloning: A Laboratory Manual. 2nd ed., Cold Spring Harbor Laboratory Press, Cold Spring Harbor, NY; Ausubel, F.M. et al. (2001) Current Protocols in Molecular Biology. Wiley & Sons, Hoboken, NJ). Examples of such methods are RT-PCR, RNAse protection assay, Northern analysis, Western analysis, ELISA, radioimmunoassay or fluorescence titration assay.

For example, an mRNA can be detected by hybridization with a labeled nucleic acid probe (DNA or RNA) and subsequent analysis using either Northern blotting or performing an RNAse protection assay. Alternatively, the respective cDNA can be prepared from cellular RNA (either total RNA or mRNA) by reverse transcription and analyzed for the absence or presence of a particular nucleic acid species by PCR amplification (i.e. RT-PCR). Proteins can be conveniently detected by using specific antibodies, which may be monoclonal or polyclonal antibodies. For visualization, the antibody may be labeled or a secondary antibody conjugated to an appropriate label may be used, which binds to the specific primary antibody. Detection may be done, for example, in a Western blot-analysis or an ELISA. Suitable labels for performing the methods of the invention include enzyme labels, radioactive labels, fluorescent labels, chromogenic labels, luminescent labels, digoxigenin, biotin, small organic molecules, metals, metal complexes, and colloidal gold.

In another preferred embodiment of the disclosure, the method further comprises:
(c) determining one or more of the following in the one or more malignant cells of the patient to be treated before administering the medicament or the combination of medicaments:
   (i) the presence or absence of one or more *ras* gene mutations;
   (ii) the presence or absence of one or more *FGFR3* gene mutations; and
   (iii) the presence or absence of one or more *PTEN* gene mutations.

In one embodiment, the method further comprises comparing results of the measurements obtained in the one or more malignant cells with those obtained in one or more control cells, i.e. non-tumorgenic cells, of a healthy subject.

The term *"ras* gene mutation", as used herein, refers to any genetic variation of a member of the *ras* gene family, i.e. the H-*ras*, K-*ras*, and *N-ras* genes, in particular of the human *ras* genes (see Land, H. et al. (1983) Science 222, 771-778). Genetic variations according to the present invention include the addition, the deletion, and the substitution of one or more nucleotides of the respective DNA sequence. Preferably, such one or more mutations give rise to an altered amino acid sequence of the respective corresponding Ras protein, which also includes the formation of truncated or extended protein variants. Such mutations may also affect protein function. For example, they may cause a (constitutive) activation of the Ras protein or may result in a dominant-negative Ras variant. Examples of such *ras* gene mutations include *inter alia* mutations affecting codons 12, 13, and 61 of human N-*ras* as well as codons 12 and 71 of human K-*ras* (cf. the references cited below).

In preferred embodiments, the one or more *ras* gene mutations cause an activation of the corresponding Ras protein (i.e. an increase of Ras protein activity). Particular preferred examples of such *ras* gene mutations comprise *inter alia* mutations at codon 61 of human N-*ras* including CAA → CGA and CAA → CAC as well as mutations at codon 12 of human K-ras including GGT → GCT and GGT → GAT (cf., e.g., Bos, J.L. et al. (1984) Nucl. Acids Res. 12, 9155-9163; Pilz, R.B. et al. (1997) Cell Growth Diff. 8, 53-59; Delgado, M.D. et al. (2000) Oncogene 19, 783-790; Chesi, M. et al. (2001) Blood 97, 729-736).

The term *FGFR3* gene mutation", as used herein, refers to any genetic variation of the fibroblast growth factor receptor 3 (*FGFR3*) gene, in particular of the human *FGFR3* gene (Ornitz, D.M., and Itoh, N. (2001) Genome Biol. 2, reviews 3005.1-3005.12). Genetic variations according to the present invention include the addition, the deletion, and the substitution of one or more nucleotides of the respective DNA sequence. Preferably, such one or more mutations give rise to an altered amino acid sequence of the respective corresponding FGFR3 protein, which also includes the formation of truncated or extended protein variants. Such mutations may also affect protein function. For example, they may cause a (constitutive) activation of the FGFR3 protein or may result in a dominant-negative FGFR3 variant. Examples of such *FGFR3* gene mutations include *inter alia* mutations affecting codons 373, 384, and 650 of the human *FGFR3* as well as a deletion of the human *FGFR3* stop codon (cf. the references cited below).

In preferred embodiments, the one or more *FGFR3* gene mutations cause an activation of the corresponding FGFR3 protein (i.e. an increase of FGFR3 protein activity). Particular preferred examples of such *FGFR3* gene mutations comprise *inter alia* mutations of human *FGFR3* at codon 373 including TAT → TGT, at codon 650 including AAG → GAG, and a deletion of the human *FGFR3* stop codon (cf., e.g., Chesi, M. et al. (1997) Nat. Genet. 16, 260-264; Chesi, M. et al. (2001) Blood 97, 729-736).

In particular preferred embodiments, the presence or absence of the one or more *FGFR3* gene mutations are determined in the concomitant presence of the t(4;14) chromosomal translocation. That is, the sequence of the *FGFR3* gene is analyzed in one or more malignant cells (such as hematopoetic cells) bearing said chromosomal translocation.

The term "t(4;14) chromosomal translocation", as used herein, denotes a karyotypically silent genetic translocation, i.e. an abnormal chromosomal rearrangement caused by the interchange of parts between non-homologous chromosomes, on human chromosome 14, in particular it denotes the t(4;14)(p16;q32) translocation, which results in a dysregulated expression of the fibroblast growth factor receptor 3 gene (*FGFR3* gene) (see, for example, Richelda, R. et al. (1997) Blood 90, 4062-4070; Chesi, M. et al. (2001) Blood 97, 729-736).

The presence of the t(4;14) chromosomal translocation in the one or more malignant cells to be analyzed may *inter alia* be determined by classical Southern analyses (cf., for example, Sambrook, J. et al. (1989), *supra*; Ausubel, F.M. et al. (2001), *supra*) or by *in situ* hybridization techniques, preferably be fluorescence *in situ* hybridization (FISH) (for review see, for example, van der Ploeg, M. (2000) Eur. J. Histochem. 44, 7-42; Levsky, J.M. and Singer, R.H. (2003) J. Cell Sci. 116, 2833-2838).

The term "*PTEN* gene mutation", as used herein, refers to any genetic variation of the *PTEN* tumor suppressor gene, in particular of the human *PTEN* gene (see, e.g., Sakai, A. et al. (1998) Blood 92, 3410-3415; Wu, H. et al. (2003) Oncogene 22, 3113-3122). Genetic variations according to the present invention include the addition, the deletion, and the substitution of one or more nucleotides of the respective DNA sequence. Preferably, such one or more mutations give rise to an altered amino acid sequence of the respective corresponding PTEN protein, which also includes the formation of truncated or extended protein variants. Such mutations may also affect protein function. For example, they may cause a (constitutive) activation of the PTEN protein or may result in a dominant-negative PTEN variant.

Preferred examples of *PTEN* gene mutations to be determined in the present invention include *inter alia* a deletion of exons 2-5 of the human *PTEN* gene, a 2 bp (base pairs) deletion and 9 bp insertion at codon 234, and a 39 bp insertion at codon 246 of the human *PTEN* gene sequence, respectively.

The respective mutations of the *ras*, *FGFR3*, and *PTEN* genes are determined by established standard laboratory methods, typically by polymerase chain reaction (PCR), restriction analyses and/or DNA sequence analyses (cf., for example, Sambrook, J. et al. (1989), *supra*; Ausubel, F.M. et al. (2001), *supra*).

Instead of or in addition to determining the absence or presence of one or more mutations of the *ras*, *FGFR3*, and/or *PTEN* genes, respectively, which all affect MAPK/ERK- and/or PI3K/AKT-signaling (reviewed, e.g., in Johnson, G.L. and Lapadat, R.(2002) Science 298, 1911-1913; Lee, J.T., and McCubrey, J.A. (2002) Leukemia 16, 486-507; Platanias, L.C. (2003) Blood 101, 4667-4679; Meier, F. et al. (2005) Front. Biosci. 10, 2986-3001; Martelli, A.M. et al. (2006) Leukemia 20, 911-928) it may also be possible within the scope of the present invention to determine the "activity status" (i.e. activation or not) of the respective signaling pathway "as such". For example, an activation of the MAPK/ERK pathway may be determined by measuring the phosphorylation of ERK-1/2 by immunochemical methods well known in the art such as Western analysis, immune precipitation, and ELISA, preferably by employing phosphorylation-specific anti-ERK-1/2 antibodies commercially available by various suppliers (for a review of immunological methods see, e.g., Coico, R. et al. (2006) Current Protocols in Immunology. Wiley & Sons, Hoboken,NJ).

In another aspect, the present invention relates to an *in vitro* method for determining the susceptibility of one or more malignant cells to a medicament or a combination of medicaments as defined in the invention, the method comprising:
(a) determining the extent of Fas receptor gene expression in the one or more malignant cells.

In a preferred embodiment, the method further comprises:
(b) determining one or more of the following in the one or more malignant cells:
   (i) the presence or absence of one or more *ras* gene mutations;
   (ii) the presence or absence of one or more *FGFR3* gene mutations; and
   (iii) the presence or absence of *PTEN* gene mutations.

In preferred embodiments, the presence or absence of the one or more *FGFR3* gene mutations is determined in the concomitant presence of the t(4;14) chromosomal translocation.

Optionally, the method further comprises:
(c) comparing the results of the measurements obtained in (a) and/or (b) with those obtained in one or more control cells.

In another aspect, the present invention relates to a tri-substituted glycerol compound as defined herein for use in the treatment of hematological malignancies. In preferred embodiments, the hematological malignancies are selected from the group consisting 15 of leukemia, lymphoma, multiple myeloma, and myelodysplastic syndrome.

The present invention further relates to a kit-of-parts comprising a combination of medicaments as defined in any one of the claims 1 to 7 for use in the treatment of hematological malignancies.

The components (parts) of the kit (i.e. the individual medicaments or active ingredients) may be administered during a combination treatment (regimen) either simultaneously, separately or sequentially (cf., for example, Sambrook, J. et al. (1989), *supra*; Ausubel, F.M. et al. (2001), *supra*).

The invention is further described by the following figures and examples, which are solely for the purpose of illustrating specific embodiments of this invention, and are not to be construed as limiting the scope of the invention in any way.

Materials used in tests below are either commercially available or easily prepared from commercially available materials by those skilled in the art.

### FIGURES

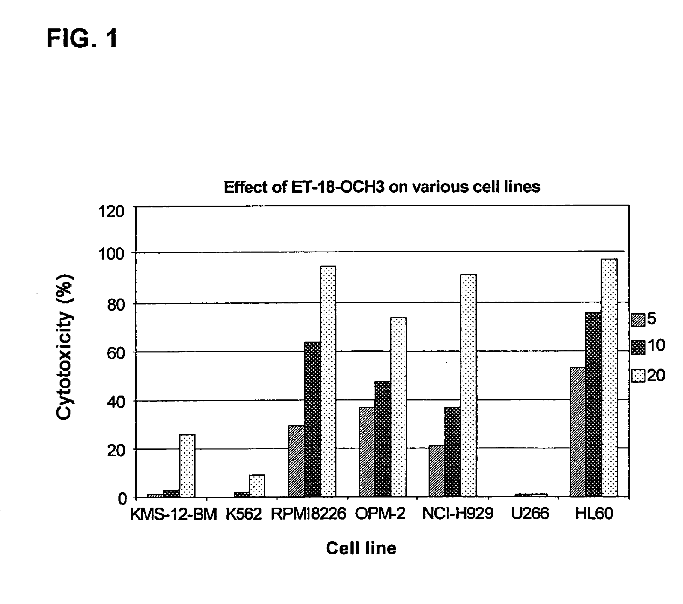

- **Figure 1**: depicts the cytotoxic effects of ET-18-OCH3 (1-*O*-octadecyl-2-*O*-methyl-glycero-3-phosphocholine) on various tumor cell lines. The following cell lines were used: KMS-12-BM, K562, RPMI8226, OPM-2, NCI-H929, U266, and HL60. The cells were grown in 96-well tissue culture plates in a volume of 200 µl to a concentration of 1 x 10⁶ cells/ml. Then, the cells were incubated for 20 h with 5 µg/ml, 10 µg/ml, and 20 µg/ml ET-18-OCH3 (final concentrations), respectively. Cell death was assessed by staining the cells with 7-amino-actinomycin D (final concentration 200 µg/ml) and analyzed via flow cytometry as described (see, e.g., Schmid, I. et al. (1994) *J. Immunol. Meth.* **170**, 145-157; Ayuk, F.A. et al. (2005) *Exp. Hematol.* **33**, 1531-1536).

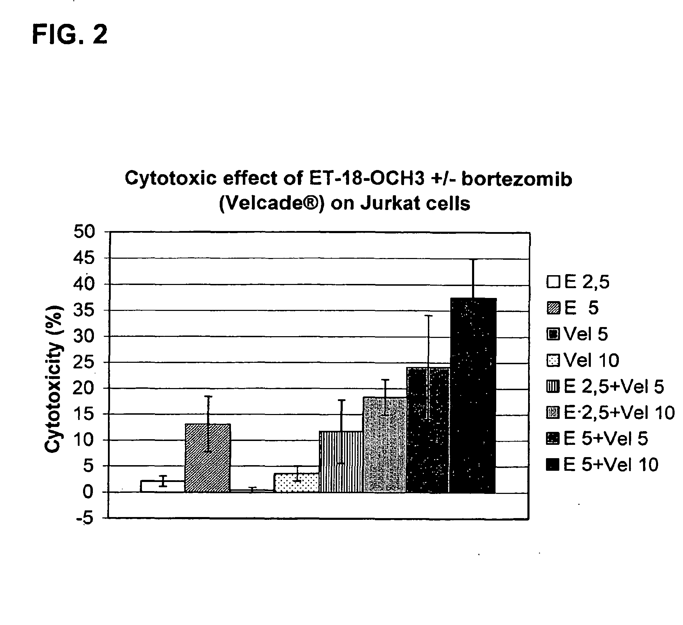

- **Figure 2**: depicts the cytotoxic effects of ET-18-OCH3 alone and in combination with bortezomib (Velcade®), an inhibitor of the 26S proteasome, on Jurkat cells. Cell culture was performed as described in Fig. 1. ET-18-OCH3 was added in a final concentration of 2.5 or 5.0 µg/ml ("E2.5" and "E5", respectively) without or in combination with 5 or 10 ng/ml bortezomib ("Vel5" and "Vel10", respectively), and the cells were incubated for 20 h. Cell death was assessed as described in Fig. 1. The results are expressed as mean ± SEM of three independent experiments.

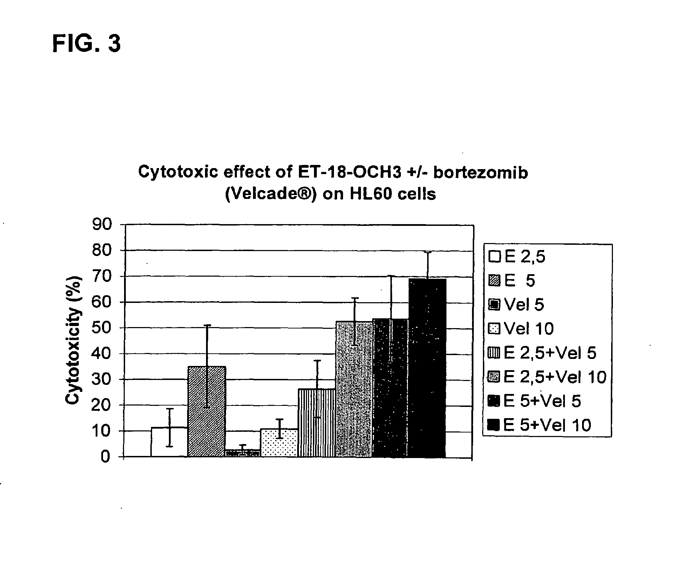

- **Figure 3**: depicts the cytotoxic effects of ET-18-OCH3 alone and in combination with bortezomib on HL60 cells. Cell culture, compound concentrations, and assay conditions were as described in Fig. 2. The results are expressed as mean ± SEM of four independent experiments.

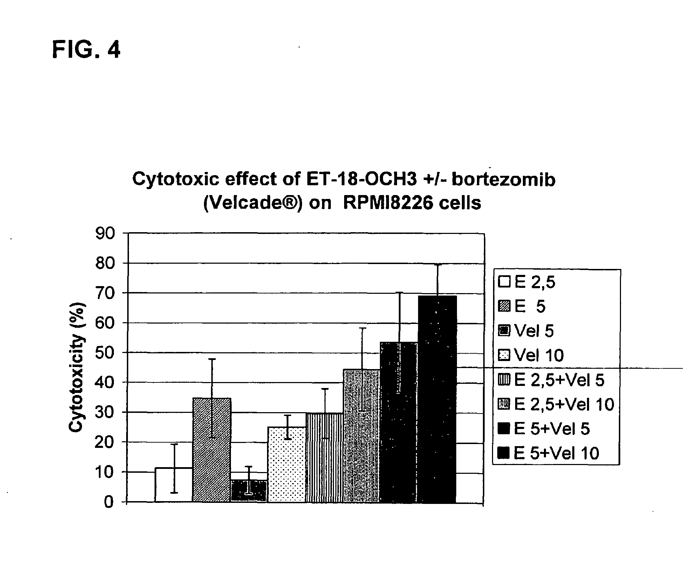

- **Figure 4**: depicts the cytotoxic effects of ET-18-OCH3 alone and in combination with bortezomib on RPMI8226. Cell culture, compound concentrations, and assay conditions were as described in Fig. 2. The results are expressed as mean ± SEM of three independent experiments.

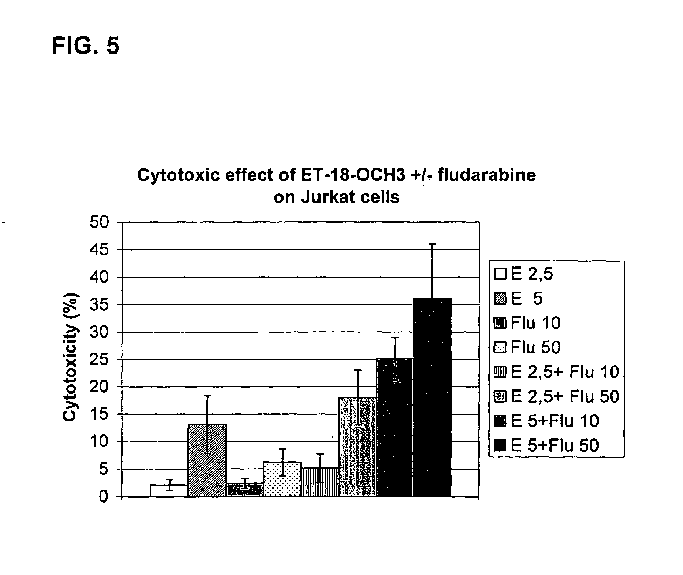

- **Figure 5**: depicts the cytotoxic effects of ET-18-OCH3 alone and in combination with fludarabine, a purine analog, on Jurkat cells. Cell culture was performed as described in Fig. 1. ET-18-OCH3 was added in a final concentration of 2.5 or 5.0 µg/ml ("E2.5" and "E5", respectively) without or in combination with 10 or 50 µM fludarabine ("Flu10" and "Flu50", respectively), and the cells were incubated for 20 h. Cell death was assessed as described in Fig. 1. The results are expressed as mean ± SEM of three independent experiments.

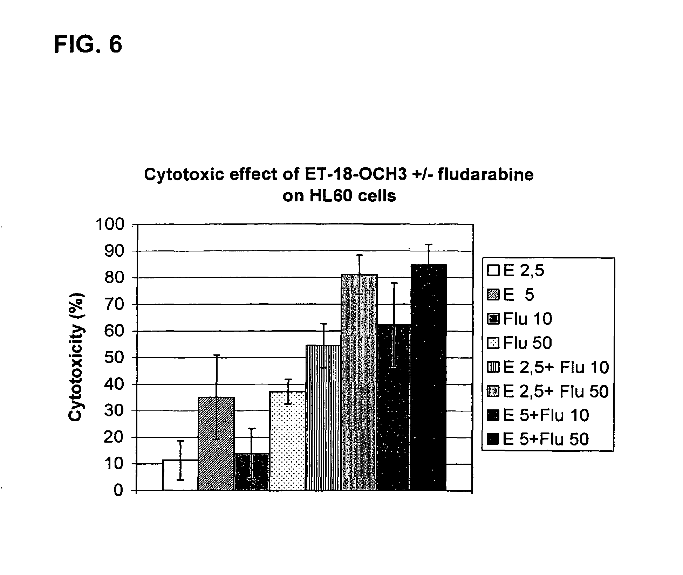

- **Figure 6**: depicts the cytotoxic effects of ET-18-OCH3 alone and in combination with fludarabine on HL60 cells. Cell culture, compound concentrations, and assay conditions were as described in Fig. 5. The results are expressed as mean ± SEM of four independent experiments.

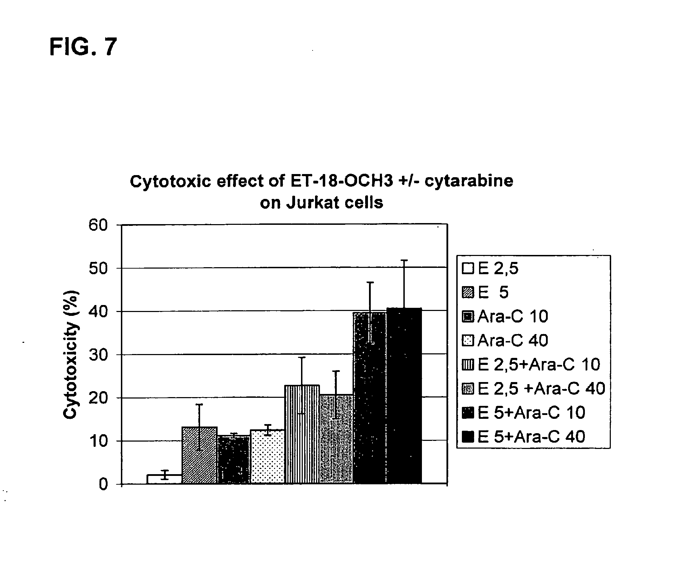

- **Figure 7**: depicts the cytotoxic effects of ET-18-OCH3 alone and in combination with cytarabine, a pyrimidine analog, on Jurkat cells. Cell culture was performed as described in Fig. 1. ET-18-OCH3 was added in a final concentration of 2.5 or 5.0 µg/ml ("E2.5" and "E5", respectively) without or in combination with 10 or 40 µM cytarabine ("Ara-C10" and "Ara-C40", respectively), and the cells were incubated for 20 h. Cell death was assessed as described in Fig. 1. The results are expressed as mean ± SEM of three independent experiments.

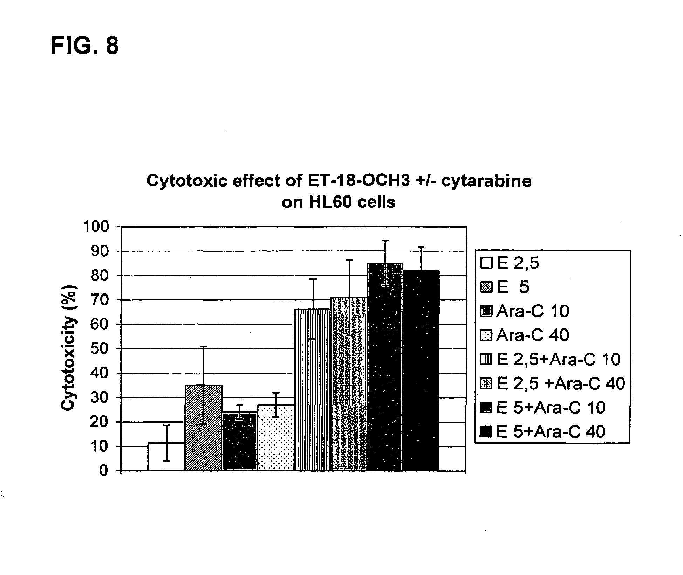

- **Figure 8**: depicts the cytotoxic effects of ET-18-OCH3 alone and in combination with cytarabine on HL60 cells. Cell culture, compound concentrations, and assay conditions were as described in Fig. 7. The results are expressed as mean ± SEM of four independent experiments.

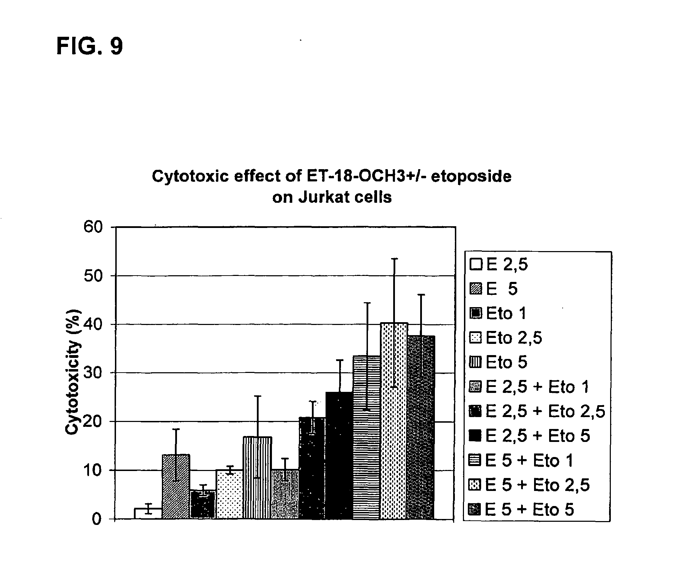

- **Figure 9**: depicts the cytotoxic effects of ET-18-OCH3 alone and in combination with etoposide, a topoisomerase II inhibitor, on Jurkat cells. Cell culture was performed as described in Fig. 1. ET-18-OCH3 was added in a final concentration of 2.5 or 5.0 µg/ml ("E2.5" and "yes", respectively) without or in combination with 1.0, 2.5 or 5.0 µM etoposide ("Eto1", "Eto2.5", and "Eto5", respectively), and the cells were incubated for 20 h. Cell death was assessed as described in Fig. 1. The results are expressed as mean ± SEM of three independent experiments.

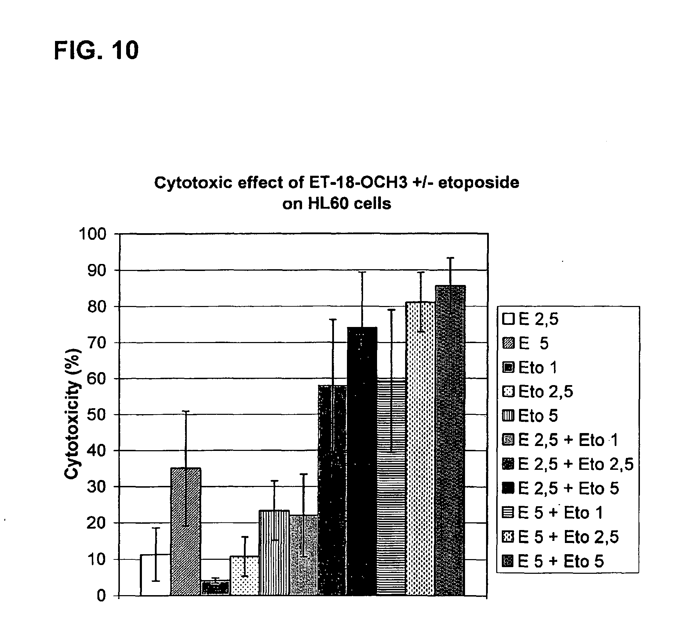

- **Figure 10**: depicts the cytotoxic effects of ET-18-OCH3 alone and in combination with etoposide on HL60 cells. Cell culture, compound concentrations, and assay conditions were as described in Fig. 9. The results are expressed as mean ± SEM of four independent experiments.

### EXAMPLES

### Reference Example 1: Determining the cytotoxic effect of 1-O-octadecyl-2-O-methyl-glycero-3-phosphocholine on various human hematological tumor cell lines

The following human cell lines were used:
1. KMS-12-BM (DMSZ no. ACC 551), bone marrow cells; myeloma/plasmacytoma; none of the genetic alterations defined herein; Chesi, M. et al. (2001), *supra.*
2. K562 (ATCC no. CCL-243); bone marrow cells; CML; none of the genetic alterations defined herein; Delgado, M.D. et al. (2001), *supra.*
3. RPMI8226 (ATCC no. CLL-155); peripheral blood cells; myeloma/plasmacytoma; K12-G>A K-*ras* gene mutation (GGT → GCT at codon 12); Chesi, M. et al. (2001), *supra.*
4. OPM-2 (ATCC no. CRL-13007); lymphoblast cells; myeloma; t(4; 14) chromosomal translocation; K650E *FGFR3* gene mutation (AAG → GAG at codon 650); Chesi, M. et al. (2001), *supra.*
5. NCI-H929 (ATCC no. CRL-9068); bone marrow cells; myeloma/plasmacytoma; t(4;14) chromosomal translocation; N13-G>D N-*ras* gene mutation (GGT → GAT at codon 13); Chesi, M. et al. (2001), *supra.*
6. U266 (ATCC no. TIB-196); peripheral blood cells; myeloma/plasmacytoma; none of the genetic alterations defined herein; Chesi, M. et al. (2001), *supra*.
7. HL60 (ATCC no. CCL-240); peripheral blood cells; AML; N61-Q>R *N-ras* gene mutation (CAA → CGA at codon 61); Bos, J.L. et al. (1984), *supra*.
8. Jurkat (ATCC no. TIB-152); peripheral blood cells; ALL; 2 bp deletion and 9 bp insertion at codon 234 in exon 7 of the *PTEN* gene (resulting in downstream stop codons); Sakai, A. et al. (1998), *supra*.

The cells were grown in 96-well tissue culture plates in a volume of 200 µl to a concentration of 1 x 10⁶ cells/ml. Then, the cells were incubated for 20 h with a final concentration of 5 µg/ml, 10 µg/ml, and 20 µg/ml 1-O-octadecyl-2-O-methyl-glycero-3-phosphocholine (ET-18-OCH3), respectively. Cell death was assessed by staining the cells with 7-amino-actinomycin D (Calbiochem, Darmstadt, Germany; final concentration 200 µg/ml) and analyzed via flow cytometry using a FACS-Scan flow cytometer (BD Biosciences, San Jose, California, USA) as described (see, e.g., Schmid, I. et al. (1994) J. Immunol. Meth. 170, 145-157; Ayuk, F.A. et al. (2005) Exp. Hematol. 33, 1531-1536).

The results obtained in a typical experiment are summarized in Fig. 1. With the exception of U266 cells, which apparently do not respond to ET-18-OCH3 at all, when exposed to ET-18-OCH3 all cell lines showed a dose-dependent effect. The application of 20 µg/ml ET-18-OCH3 resulted in almost complete cell death in RPMI8226, NCI-H929, and HL60 cells, respectively. In OPM-2 cells about 75% of the cells died upon exposure to 20 µg/ml ET-18-OCH3. Interestingly, in those cell lines harboring none of the genetic alterations defined herein, namely the t(4;14) chromosomal translocation, *ras*, *FGFR3*, and *PTEN* gene mutations, respectively (i.e. in KMS-12-BM, K562, and U266), the cytotoxicity is significantly reduced or even almost abolished. These results demonstrate that the presence of one or more of the above genetic alterations is a strong indication for the susceptibility of a given cell to the treatment with ET-18-OCH3.

### Example 2: Determining the cytotoxic effect of 1-O-octadecyl-2-O-methyl-glycero-3-phophocholine in combination with different anti-neoplastic agents on various cell lines

The following cytostatic drugs were used: bortezomib (Velcade®; Janssen Cilag, Neuss, Germany), an inhibitor of the 26S proteasome that is commonly used for the treatment of multiple myeloma; fludarabine (Sigma, St. Louis, USA), a purine analog mainly used in the treatment of AML and CLL; cytarabine (also referred to as "cytosine arabinoside" or "Ara-C"; Sigma, St. Louis, USA), a pyrimidine analog mainly used in the treatment of AML and non-Hodgkin lymphomas; and etoposide (Sigma, St. Louis, USA), an inhibitor of topoisomerase II that is commonly used in the treatment of lymphomas.

The cells were again grown in 96-well tissue culture plates in a volume of 200 µl to a concentration of 1 x 10⁶ cells/ml. Then, the cells were incubated for 20 h with the respective drugs. The following final concentrations were used (in general, the concentrations employed resulted in less than 50% cytotoxicity when administered alone): 2.5 µg/ml and 5.0 µg/ml 1-*O*-octadecyl-2-*O*-methyl-glycero-3-phosphocholine (ET-18-OCH3); 5 ng/ml and 10 ng/ml bortezomib; 10 µM and 50 µM fludarabine; 10 µM and 40 µM cytarabine; and 1 µM, 2.5 µM, and 10 µM etoposide, respectively. The cytotoxic effect of ET-18-OCH3 in combination with bortezomib was determined in Jurkat, HL60 cells, and RPMI8226 cell, whereas the cytotoxic effect of ET-18-OCH3 in combination with cytarabine, fludarabine and etoposide, respectively, in Jurkat and HL60 cells. Cell death was assessed as described in example 1.

The results obtained are summarized in the following Tables 1 to 4 (the data represent means ± SEM of 3 (Jurkat cells and RPMI8226 cells) or 4 independent experiments (HL60 cells):

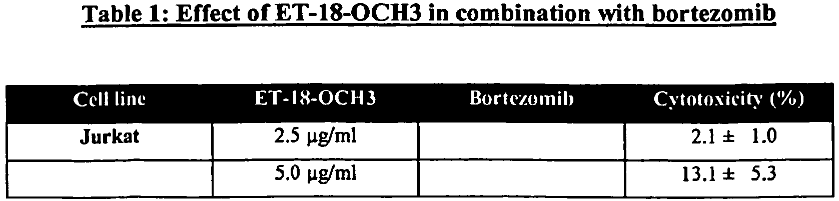

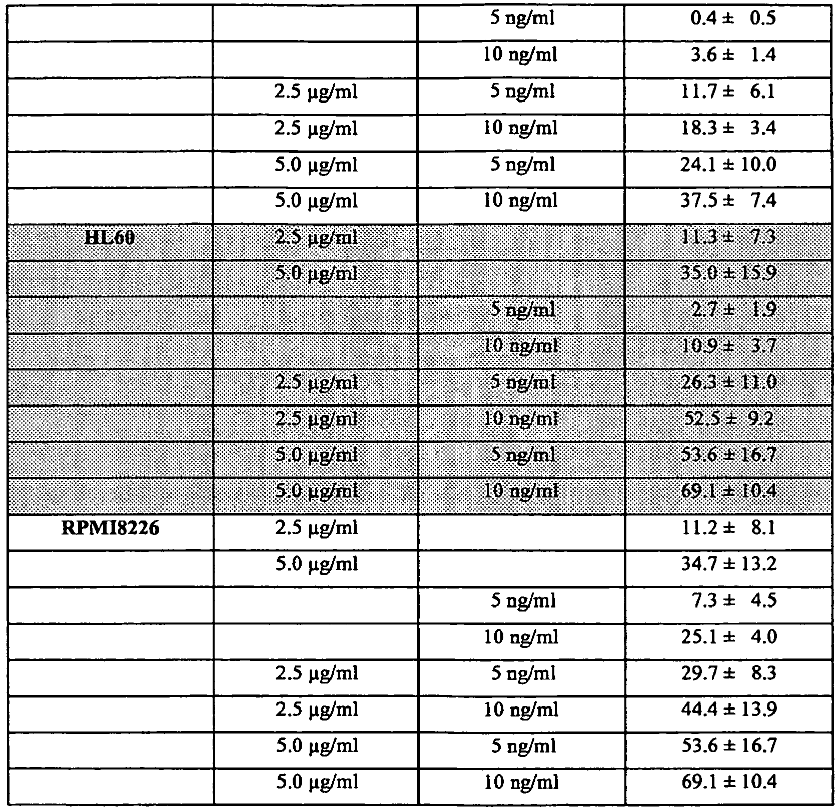

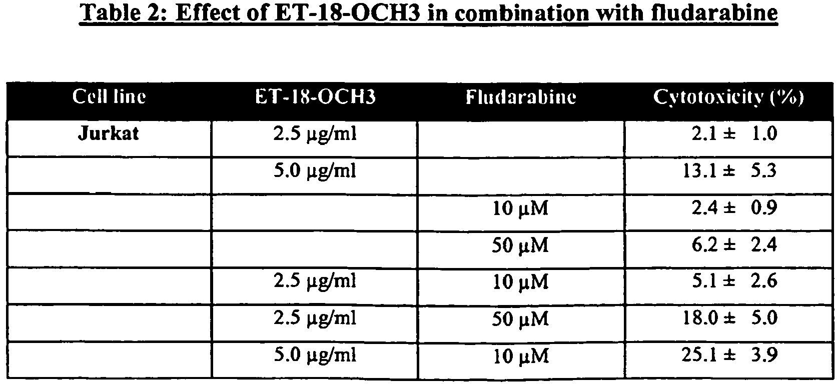

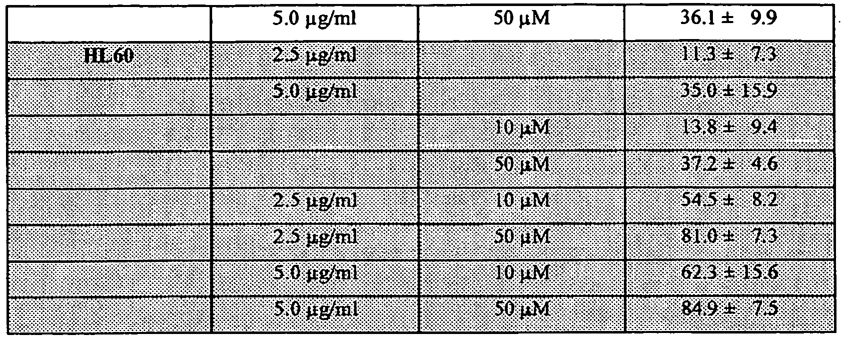

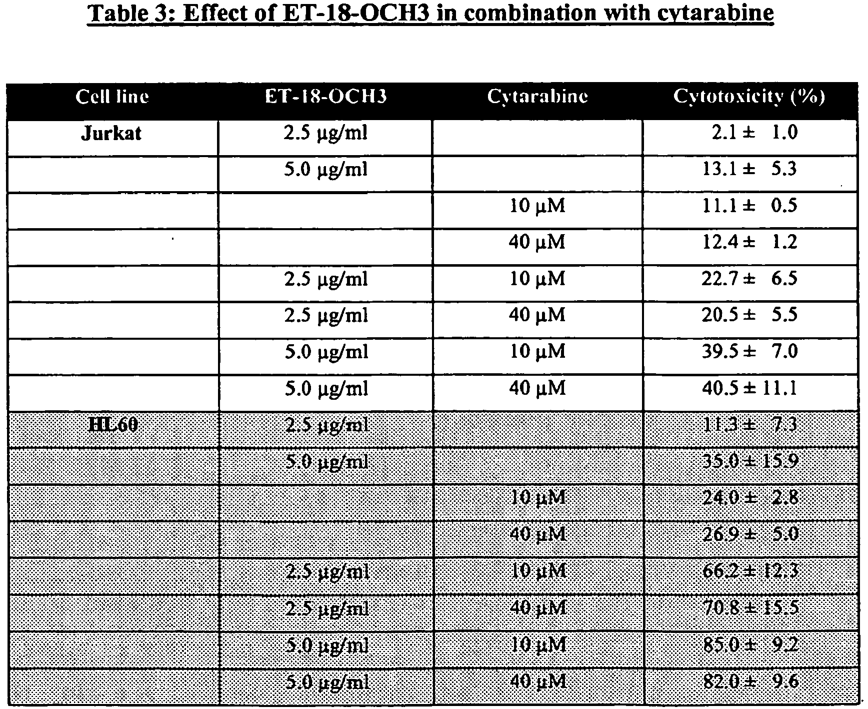

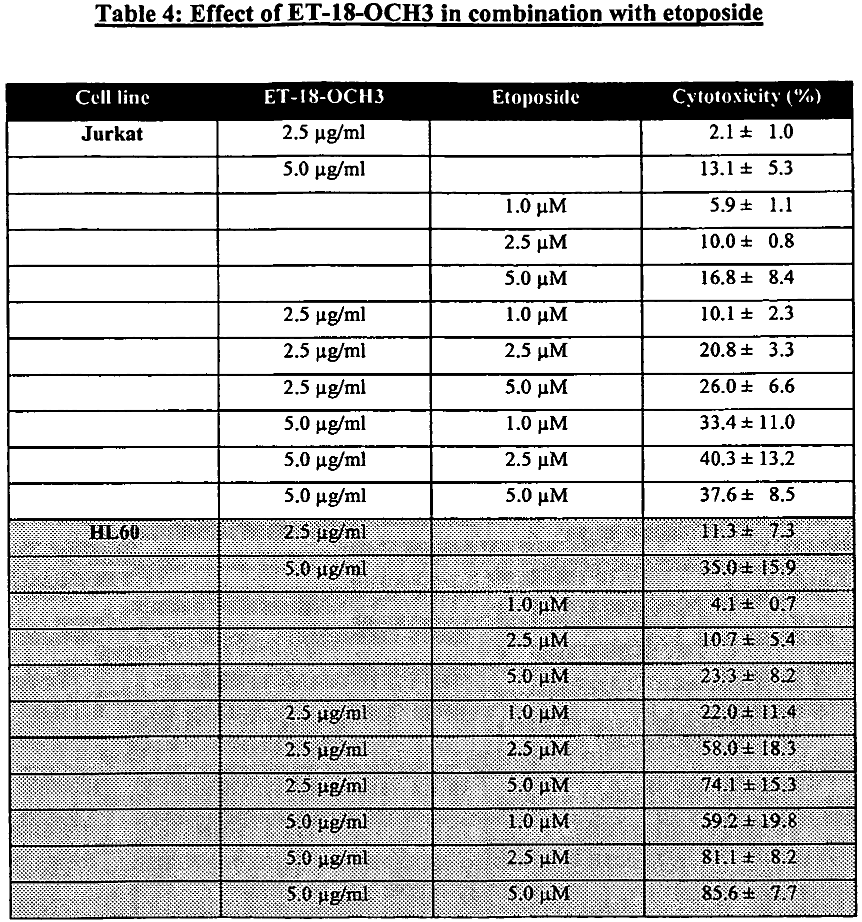

From the above results it is apparent that an adjunction of 1-*O*-octadecyl-2-*O-*methyl-glycero-3-phosphocholine (ET-18-OCH3) with any of the anti-neoplastic agents tested was giving rise to an improved cytotoxic effect in either of the cell limes employed. Importantly, the different combinations of active ingredients acted not only in an additive but in a synergistic manner. Thus, this synergistic mode of action allows for the administration of reduced drug doses in order to achieve the same pharmaceutical efficacy as compared to an individual administration, which will again reduce the risk of the occurrence of adverse side effects.

The present invention illustratively described herein may suitably be practiced in the absence of any element or elements, limitation or limitations, not specifically disclosed herein. Thus, for example, the terms "comprising", "including", "containing", etc. shall be read expansively and without limitation.

## Claims

1. Use of a tri-substituted glycerol compound according to formula (I) 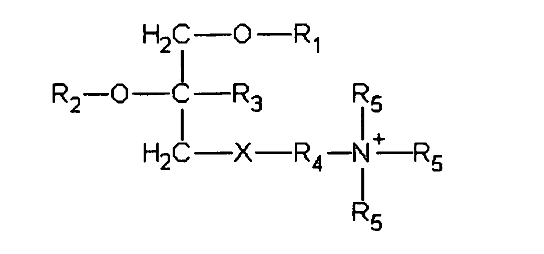 or an enantiomer or diastereomer or a pharmaceutically acceptable salt thereof and at least one pharmaceutically acceptable excipient for the manufacture of a medicament for the treatment of hematological malignancies, wherein
X is selected from the group consisting of phosphate and sulfate;
R₁ is selected from the group consisting of C₁₆- C₂₀ alkyl;
R₂ is selected from the group consisting of C₁-C₃ alkyl and C₁-C₃ hydroxyalkyl; R₃ is selected from the group consisting of hydrogen and C₁-C₃ alkyl;
R₄ is selected from the group consisting of C₁-C₃ alkyl and C₃-C₆ cycloalkyl; and R₅ is selected from the group consisting of hydrogen and methyl;
and wherein the medicament is a pharmaceutical solid dosage form for oral administration and is used in combination with at least one other medicament comprising one or more additional active ingredient/s which is/are selected from the group consisting of antimetabolites, plant alkaloids, inhibitors of topoisomerase II and proteasome inhibitors.

2. The use according to claim 1, wherein X is phosphate, R₁ is -(CH₂)₁₇-CH₃, R₂ is CH₃, R₃ is H, R₄ is -(CH₂)₂-, and R₅ is CH₃.

3. The use according to claims 1 or 2, wherein the pharmaceutical solid dosage form is selected from the group consisting of tablets, pills, capsules, and granules.

4. The use according to claim 3, wherein the medicament is an enteric dosage form.

5. The use according to any of claims 1 to 4, wherein the hematological malignancies are selected from the group consisting of leukemia, lymphoma, multiple myeloma, and myelodysplastic syndrome, with the leukemia preferably being selected from the group consisting of acute myeloid leukemia, acute lymphoid leukemia, chronic myeloid leukemia, and chronic lymphoid leukemia; or with the lymphoma preferably being selected from the group consisting of Hodgkin lymphoma and non-Hodgkin lymphoma.

6. The use according to any of claims 1-5, wherein the antimetabolites are selected from the group consisting of methotrexate, purine analogs, and pyrimidine analogs.

7. The use according to claims 1 to 6, wherein the other medicament is selected from the group consisting of methotrexate, fludarabine, clofarabine, pentostatine, cladribine, cytarabine, decitabine, vincristine, vinblastine, etoposide, teniposide and bortezomib.

8. Combination of medicaments as defined in any of claims 1 to 7 for use in the treatment of hematological malignancies, wherein the hematological malignancies are preferably selected from the group consisting of leukemia, lymphoma, multiple myeloma, and myelodysplastic syndrome.

9. *In vitro* method for determining the susceptibility of one or more malignant cells to a combination of medicaments as defined in any of claims 1 to 7, the method comprising:
(a) determining the extent of Fas receptor gene expression in the one or more malignant cells.

10. The *in vitro* method according to claim 9, further comprising:
(b) determining one or more of the following in the one or more malignant cells:
(i) the presence or absence of one or more *ras* gene mutations;
(ii) the presence or absence of one or more *FGFR3* gene mutations; and
(iii) the presence or absence of one or more *PTEN* gene mutations,
wherein the presence or absence of the one or more *FGFR3* gene mutations is preferably determined in the concomitant presence of the t(4;14) chromosomal translocation.

11. The *in vitro* method according to claim 9 or 10, further comprising:
(c) comparing the results of the measurements obtained in (a) and/or (b) with those obtained in one or more control cells.

12. Kit-of-parts, comprising a combination of medicaments as defined in any of claims 1 to 7.

## Patentansprüche

1. Verwendung einer trisubstituierten Glycerolverbindung gemäß der Formel (I) 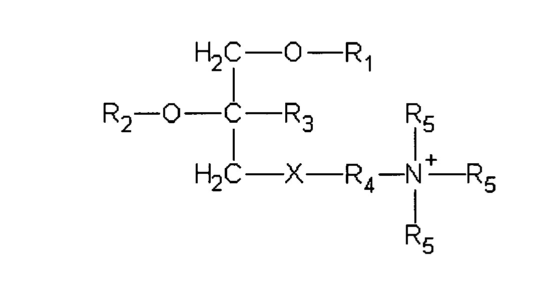 oder eines Enantiomers oder Diastereomers oder eines pharmazeutisch akzeptablen Salzes davon und mindestens eines pharmazeutisch akzeptablen Hilfsstoffs zur Herstellung eines Medikaments zur Behandlung von bösartigen hämatologischen Tumoren, wobei
X aus der Gruppe bestehend aus Phosphat und Sulfat ausgewählt ist; R₁ aus der Gruppe bestehend aus C₁₆-C₂₀-Alkyl ausgewählt ist;
R₂ aus der Gruppe bestehend aus C₁-C₃-Alkyl und C₁-C₃-Hydroxyalkyl ausgewählt ist;
R₃ aus der Gruppe bestehend aus Wasserstoff und C₁-C₃-Alkyl ausgewählt ist;
R₄ aus der Gruppe bestehend aus C₁-C₃-Alkyl und C₃-C₆-Cycloalkyl ausgewählt ist; und
R₅ aus der Gruppe bestehend aus Wasserstoff und Methyl ausgewählt ist;
und wobei das Medikament eine feste pharmazeutische Dosierungsform zur oralen Verabreichung ist und in Kombination mit mindestens einem weiteren Medikament verwendet wird, das einen oder mehrere zusätzliche aktive Inhaltsstoff(e) umfasst, der/die ausgewählt ist/sind aus der Gruppe bestehend aus Antimetaboliten, Pflanzenalkaloiden, Inhibitoren von Topoisomerase II und Proteasominhibitoren.

2. Verwendung nach Anspruch 1, wobei X Phosphat ist, R₁ -(CH₂)₁₇-CH₃ ist, R₂ CH₃ ist, R₃ H ist, R₄ -(CH₂)₂- ist und R₅ CH₃ ist.

3. Verwendung nach Anspruch 1 oder 2, wobei die feste pharmazeutische Dosierform ausgewählt ist aus der Gruppe bestehend aus Tabletten, Pillen, Kapseln und Granulat.

4. Verwendung nach Anspruch 3, wobei das Medikament eine enterische Dosierungsform ist.

5. Verwendung nach einem der Ansprüche 1-4, wobei der bösartige hämatologische Tumor ausgewählt ist aus der Gruppe bestehend aus Leukämie, Lymphom, multiplem Myelom, und myelodysplastischem Syndrom, wobei die Leukämie bevorzugt ausgewählt ist aus der Gruppe bestehend aus akuter myeloider Leukämie, akuter lymphoider Leukämie, chronischer myeloider Leukämie und chronischer lymphoider Leukämie; oder wobei das Lymphom bevorzugt ausgewählt ist aus der Gruppe bestehend aus Hodgkin-Lymphom und Nicht-Hodgkin-Lymphom.

6. Verwendung nach einem der Ansprüche 1-5, wobei die Antimetabolite ausgewählt sind aus der Gruppe bestehend aus Methotrexat, Purinanaloga und Pyrimidinanaloga.

7. Verwendung nach einem der Ansprüche 1-6, wobei das andere Medikament ausgewählt ist aus der Gruppe bestehend aus Methrotrexat, Fludarabin, Clofarabin, Pentostatin, Cladribin, Cytarabin, Decitabin, Vincristin, Vinblastin, Etoposid, Teniposid und Bortezomib.

8. Kombination von Medikamenten wie definiert in einem der Ansprüche 1-7 zur Verwendung in der Behandlung von bösartigen hämatologischen Tumoren, wobei die bösartigen hämatologischen Tumoren bevorzugt ausgewählt sind aus der Gruppe bestehend aus Leukämie, Lymphom, multiplem Myelom, und myelodysplastischem Syndrom.

9. *In vitro*-Verfahren zur Bestimmung der Empfänglichkeit von einer oder mehreren bösartigen Zellen gegenüber einer Kombination von Medikamenten wie definiert in einem der Ansprüche 1-7 umfassend:
(a) Bestimmen des Grads der Fas-Rezeptorgenexpression in der einen oder den mehreren bösartigen Zellen.

10. *In vitro*-Verfahren nach Anspruch 9, weiter umfassend:
(b) Bestimmen eines oder mehrerer der folgenden in der einen oder den mehreren bösartigen Zellen:
(i) die Gegenwart oder Abwesenheit von einer oder mehreren *ras-*Genmutationen;
(ii) die Gegenwart oder Abwesenheit von einer oder mehreren *FGFR3*-Genmutationen; und
(iii) die Gegenwart oder Abwesenheit von einer oder mehreren *PTEN*-Genmutationen;
wobei die Gegenwart oder Abwesenheit der einen oder mehreren *FGFR3*-Genmutationen bevorzugt bestimmt wird in der gleichzeitigen Anwesenheit einer t(4;14) chromosomalen Translokation.

11. *In vitro*-Verfahren nach Anspruch 9 oder 10, weiter umfassend:
(c) Vergleichen der Ergebnisse der Messungen in (a) und/oder (b) mit denen, die mit einer oder mehreren Kontrollzellen erhalten wurden.

12. Kit umfassend eine Kombination von Medikamenten wie definiert in einem der Ansprüche 1-7

## Revendications

1. Utilisation d'un composé de glycérol trisubstitué selon la formule (I) 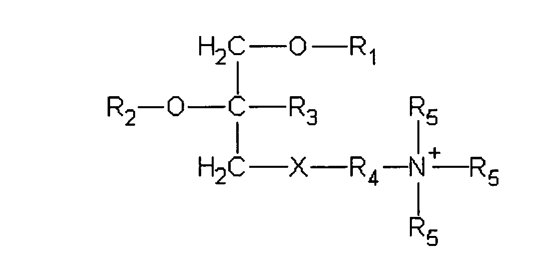 ou d'un énantiomère ou diastéréomère ou d'un sel pharmaceutiquement acceptable de ceux-ci et d'au moins un excipient pharmaceutiquement acceptable pour la fabrication d'un médicament pour le traitement de malignités hématologiques, où
X est sélectionné dans le groupe constitué par le phosphate et le sulfate ;
R₁ est sélectionné dans le groupe constitué par l'alkyle de C₁₆-C₂₀ ;
R₂ est sélectionné dans le groupe constitué par l'alkyle de C₁-C₃ et l'hydroxyalkyle de C₁-C₃ ;
R₃ est sélectionné dans le groupe constitué par l'hydrogène et l'alkyle de C₁-C₃ ; R₄ est sélectionné dans le groupe constitué par l'alkyle de C₁-C₃ et le cycloalkyle de C₃-C₆ ; et
R₅ est sélectionné dans le groupe constitué par l'hydrogène et le méthyle ;
et où le médicament est une forme galénique solide pharmaceutique destinée à être administrée par voie orale et où il est utilisé en combinaison avec au moins un autre médicament comprenant un ou plusieurs ingrédients actifs supplémentaires qui sont sélectionnés dans le groupe constitué par les antimétabolites, les alcaloïdes végétaux, les inhibiteurs de topoisomérase II et les inhibiteurs de protéasome.

2. L'utilisation selon la revendication 1, où X est du phosphate, R₁ est -(CH₂)₁₇-CH₃, R₂ est CH₃, R₃ est H, R₄ est -(CH₂)₂-, et R₅ est CH₃.

3. L'utilisation selon les revendications 1 ou 2, où la forme galénique solide pharmaceutique est sélectionnée dans le groupe constitué par les comprimés, les pilules, les capsules et les granules.

4. L'utilisation selon la revendication 3, où le médicament est une forme galénique entérique.

5. L'utilisation selon l'une quelconque des revendications 1 à 4, où les malignités hématologiques sont sélectionnées dans le groupe constitué par la leucémie, le lymphome, le myélome multiple et le syndrome myélodysplasique, la leucémie étant de préférence sélectionnée dans le groupe constitué par la leucémie myéloïde aiguë, la leucémie lymphoïde aiguë, la leucémie myéloïde chronique et la leucémie lymphoïde chronique ; ou le lymphome étant de préférence sélectionné dans le groupe constitué par le lymphome hodgkinien et le lymphome non hodgkinien.

6. L'utilisation selon l'une quelconque des revendications 1 à 5, où les antimétabolites sont sélectionnés dans le groupe constitué par le méthotrexate, les analogues de purine et les analogues de pyrimidine.

7. L'utilisation selon les revendications 1 à 6, où l'autre médicament est sélectionné dans le groupe constitué par le méthotrexate, la fludarabine, la clofarabine, la pentostatine, la cladribine, la cytarabine, la décitabine, la vincristine, la vinblastine, l'étoposide, le téniposide et le bortézomib.

8. Combinaison de médicaments telle que définie dans l'une quelconque des revendications 1 à 7, destinée à être utilisée dans le traitement de malignités hématologiques, où les malignités hématologiques sont de préférence sélectionnées dans le groupe constitué par la leucémie, le lymphome, le myélome multiple et le syndrome myélodysplasique.

9. Procédé *in vitro* de détermination de la réceptivité d'une ou de plusieurs cellules malignes à une combinaison de médicaments telle que définie dans l'une quelconque des revendications 1 à 7, le procédé comprenant :
(a) la détermination de l'étendue de l'expression du gène du récepteur Fas dans l'une ou les multiples cellules malignes.

10. Le procédé *in vitro* selon la revendication 9, comprenant en outre :
(b) la détermination d'un ou de plusieurs des éléments suivants dans l'une ou les multiples cellules malignes :
(i) la présence ou l'absence d'une ou plusieurs mutations du gène *ras ;*
(ii) la présence ou l'absence d'une ou plusieurs mutations du gène *FGFR3*;
(iii) la présence ou l'absence d'une ou plusieurs mutations de gène *PTEN* ;
où la présence ou l'absence de l'une ou plusieurs mutations du gène *FGFR3* est de préférence déterminée dans la présence concomitante de la translocation chromosomique t(4 ; 14).

11. Le procédé *in vitro* selon la revendication 9 ou 10, comprenant en outre :
(c) la comparaison des résultats de mesures obtenus dans (a) et/ou (b) avec ceux obtenus dans une ou plusieurs cellules de contrôle.

12. Kit d'éléments comprenant une combinaison de médicaments telle que définie dans l'une quelconque des revendications 1 à 7.
